# FASCICULE DE BREVET EUROPEEN

(11) **EP 0 878 466 B1**
(45) Date de publication et mention de la délivrance du brevet: **13.06.2001**
(21) Numéro de dépôt: 98401031.4
(22) Date de dépôt: 28.04.1998
(51) Int. Cl.: C07C 315/06, C07C 317/04

(54) **Procédé de purification du diméthylsulfoxide (DMSO)**
Verfahren zur Reinigung von Dimethylsulfoxid (DMSO)
Process for the purification de dimethylsulphoxide (DMSO)

(30) Priorité: 15.05.1997 FR 9705967
(43) Date de publication de la demande: 18.11.1998
(73) Titulaire: ELF AQUITAINE EXPLORATION PRODUCTION FRANCE, 92400 Courbevoie (FR)
(72) Inventeur: Commarieu, Annie, 92400 Courbevoie (FR); Humblot, Francis, 64300 Lanneplaa (FR)
(74) Mandataire: Leboulenger, Jean

(56) Documents cités:
- FR-A- 2 014 385
- A.M. PHIPPS: "Anion exchange in dimethyl sulphoxide" ANALYTICAL CHEMISTRY, vol. 40, no. 12, octobre 1968, pages 1769-1773, XP000563242 WASHINGTON, DC, US
- T. CHAUDRON, ET AL.: "La purification du diméthylsulfoxyde: critères de pureté" CHIMIE ANALYTIQUE, vol. 53, no. 5, mai 1971, pages 310-314, XP002004909 PARIS, FR

## Description

La présente invention concerne un procédé de purification de diméthylsulfoxyde (DMSO).

Le DMSO disponible actuellement sur le marché est un produit ayant déjà une bonne pureté. Ses spécifications commerciales sont généralement :

| | |
|---|---|
| pureté | ≥ 99,7 % par chromatographie |
| acidité | ≤ 0,04 mg KOH/g par potentiométrie |
| point de cristallisation | ≤ 18,1°C |
| aspect visuel | ≤ limpide |
| teneur en eau | ≤ 0,15 % |
| couleur (APHA) | ≤ 10 |

Le brevet FR 2 014 385 décrit un procédé de préparation de DMSO purifié mettant en oeuvre une résine échangeuse d'ions. Dans les deux exemples de ce brevet, on utilise une résine fortement basique du type Amberlite IR-A 400 ou Merck III, pour traiter des mélanges ternaires diméthylsulfure/DMSO/acide sulfurique à 10 %. En fait, dans ce procédé connu, la purification semble essentiellement apportée par une distillation fractionnée d'une solution aqueuse de DMSO traitée auparavant par un échangeur d'anions.

On a effectué maintenant des analyses de traces métalliques sur plusieurs échantillons de DMSO commercial, de différentes provenances. Ces analyses sont rapportées dans le tableau 1.

Les concentrations en sodium, fer, potassium, calcium, chrome, cuivre, nickel et zinc ont été mesurées par ICP (spectrométrie d'émission atomique-torche à plasma, appareil Perkin Elmer, modèle Optima 3000) et sont exprimées en ppb (1 ppb = 1 partie en poids par milliard = 1 µg par kg).

La liste des éléments métalliques figurant dans le tableau 1 n'est pas exhaustive quant aux éléments métalliques présents dans ces échantillons.

**TABLEAU 1**

| Echantillon | Cations métalliques | | | | | | | |
|---|---|---|---|---|---|---|---|---|
| | Na | Fe | K | Ca | Cr | Cu | Ni | Zn |
| 1 | 40 | 13 | 60 | 20 | 2 | 10 | 8 | 10 |
| 2 | 39 | 60 | 3 | 13 | 13 | < 2 | 18 | 3 |
| 3 | 30 | 40 | 3 | 20 | 12 | < 2 | 15 | 3 |
| 4 | 30 | 40 | 3 | 14 | 13 | < 2 | 15 | 3 |
| 5 | 30 | < 1 | 20 | 25 | < 2 | < 2 | < 3 | < 3 |
| 6 | 70 | 90 | 65 | 55 | 15 | 2 | 25 | 60 |
| limite de détection | 2 | 1 | 3 | 2 | 2 | 2 | 3 | 3 |

Pour certaines applications, comme par exemple en électronique ou en pharmacie, les DMSO analysés ci-dessus contiennent trop d'impuretés métalliques. En général, un DMSO contenant moins de 10 ppb de chaque contaminant métallique, alcalin et alcalino-terreux, serait nécessaire pour la plupart des utilisations dans les deux domaines techniques précités.

Le but de la présente invention est de trouver un procédé de purification du DMSO commercial ayant déjà une bonne pureté, mais cette dernière étant cependant insuffisante pour certaines applications.

L'échange d'ions par la mise en oeuvre de résines est une technique très utilisée pour les milieux aqueux et permet notamment l'obtention d'eau déionisée. L'échange d'anions en milieu DMSO liquide à faible teneur en eau a déjà été réalisé par Alan M. Phipps, Anal. Chem. 40(12) pp. 1769-1773, 1968, dans le but de mesurer les quantités d'anions fixés sur la résine dans des conditions expérimentales s'approchant de l'équilibre thermodynamique.

L'invention a maintenant pour objet un procédé de purification d'un diméthylsulfoxyde ayant une teneur en eau inférieure ou égale à 0,15 % en poids par rapport au poids total pour en diminuer la teneur en cations métalliques, alcalins et alcalino-terreux, caractérisé en ce qu'il consiste essentiellement à mettre le DMSO à purifier en contact avec au moins deux résines échangeuses de cations dont l'une au moins est une résine de type sulfonique, l'autre (ou les autres) pouvant être de type chélatant.

Une purification très efficace du DMSO quasi anhydre est obtenue par l'emploi conjoint d'une résine de type chélatante possédant par exemple des groupements aminophosphonique ou iminodiacétique, efficace pour échanger le fer et les métaux de charge multiple (Mⁿ⁺, n = 2, 3 et 4) et d'une résine de type sulfonique, efficace pour échanger le sodium et les ions monochargés.

De préférence, les résines cationiques utilisées sont à base d'un copolymère polystyrène-divinylbenzène. En effet, ces résines ont un squelette résistant aux attaques chimiques et, en particulier, elles ne se dissolvent pas dans le DMSO.

La mise en contact du DMSO à purifier avec les résines a lieu à une température allant de 18,45°C (point de fusion du DMSO) à 120°C (température limite de stabilité thermique des résines). Cette température est avantageusement comprise entre 19 et 80°C, de préférence entre 20 et 50°C.

Le DMSO à purifier peut être mis en contact avec un mélange des différentes résines ou successivement avec chacune des différentes résines.

L'opération peut être réalisée en discontinu (batch) ou en continu dans les conditions et appareillages bien connus de l'homme du métier. La séparation du DMSO purifié d'avec les résines peut se faire par tout moyen connu approprié, notamment par filtration, percolation ou centrifugation.

Pour définir la qualité du DMSO susceptible d'être obtenu purifié par le procédé selon l'invention, le fer et le sodium ont été retenus comme éléments traceurs et indicateurs de la teneur générale en cations métalliques, alcalins et alcalino-terreux.

Ce DMSO purifié se caractérise en ce qu'il comporte une teneur en cation Fe inférieure ou égale à 1 ppb et une teneur en cation Na inférieure ou égale à 2 ppb, limites respectives de détection de la méthode d'analyse par spectrométrie d'émission atomique-torche à plasma.

L'invention sera mieux comprise à l'aide de la partie expérimentale suivante décrivant un exemple de réalisation de la présente invention.

### Partie expérimentale

### I. Méthode d'analyse :

Pour analyser les traces de métaux dans le DMSO, on a utilisé l'ICP (spectrométrie d'émission atomique-torche à plasma) : l'échantillon est introduit dans une torche plasma, les différents éléments présents sont excités et émettent des photons dont l'énergie est caractéristique de l'élément puisqu'elle est définie par la structure électronique de l'élément considéré. On a utilisé en routine un appareil Perkin Elmer (modèle Optima 3000 DV).

### II. Méthodologie :

Principe : les traces métalliques sont sous forme Mⁿ⁺. Par passage du DMSO sur deux résines échangeuses de cations, elles-mêmes sous forme H⁺, on substitue les ions Mⁿ⁺ en solution par n.H⁺.

### III. Essai :

Principe : par souci de simplifier les analyses, le sodium et le fer ont été choisis comme traceurs représentatifs de l'ensemble des impuretés métalliques contenues dans le DMSO.

Le sodium est caractéristique de la pollution atmosphérique et accidentelle (poussières, environnement) et le fer est caractéristique de la pollution pouvant provenir du process (unité en acier inox).

Du DMSO dopé à 1000 ppb de fer et 1000 ppb de sodium est mis en contact successivement avec deux résines échangeuses de cations, sous forme H⁺ (2 g de chaque résine pour 100 g de DMSO) à 25°C. Après un premier échange cationique avec une résine de type chélatant, le DMSO est séparé du solide par filtration sur un fritté en polyéthylène de porosité 70 µm, puis il est mis en contact avec une résine de type sulfonique. Des échantillons de DMSO sont prélevés au cours du temps durant chacune des étapes pour suivre les évolutions de concentrations en fer et sodium.

Comme résine de type sulfonique, on a utilisé la résine Amberlyst® 35 commercialisée par Rohm & Haas, et comme résine chélatante, la résine aminophosphonique S 940 de la Société Purolite. Ces résines ont au préalable été traitées pour obtenir la forme H⁺ de la manière suivante : au travers de 90 ml de résine, on fait passer 540 ml d'HCI 5 % à un débit constant et tel que l'opération dure 30 à 45 minutes. Après avoir été rincée à l'eau déionisée jusqu'à neutralité de l'eau sortante, la résine est séchée par mise en suspension dans du méthanol et évaporation sous vide à l'évaporateur rotatif (90°C, 2000 Pa) jusqu'à observation d'un poids constant.

Le tableau 2 rassemble les teneurs en fer et sodium en fonction du temps pour le premier et le second échange.

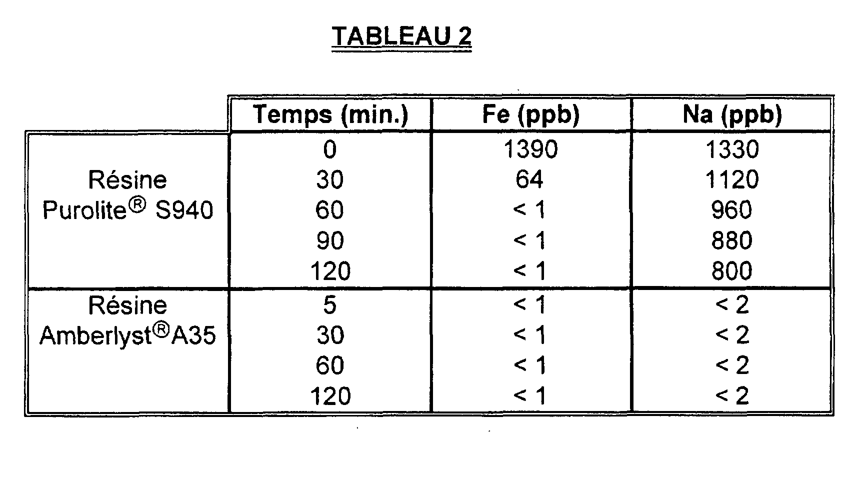

## Revendications

1. Procédé de purification d'un diméthylsulfoxyde (DMSO) ayant une teneur en eau inférieure ou égale à 0,15 % en poids par rapport au poids total pour en diminuer la teneur en cations métalliques, alcalins et alcalino-terreux, caractérisé en ce qu'il consiste à mettre le DMSO à purifier en contact avec au moins deux résines échangeuses de cations dont l'une au moins est une résine de type sulfonique, l'autre (ou les autres) étant de type chélatant, ces résines étant employées sous la forme acide ou ammonium.

2. Procédé selon la revendication 1, caractérisé en ce que chaque résine est à base d'un copolymère polystyrène-divinylbenzène.

3. Procédé selon la revendication 1 ou 2, caractérisé en ce que la mise en contact du DMSO à purifier avec les résines échangeuses a lieu à une température de 19 à 80°C.

4. Procédé selon la revendication 3, caractérisé en ce que la température est comprise entre 20 et 50°C.

## Patentansprüche

1. Verfahren zur Reinigung von Dimethylsulfoxid (DMSO) mit einem Wassergehalt, der kleiner oder gleich 0,15 Gew.-% bezogen auf das Gesamtgewicht ist, zur Verringerung seines Gehalts an Metall-, Alkali- und Erdalkalikationen, dadurch gekennzeichnet, daß es darin besteht, das zu reinigende DMSO mit mindestens zwei Kationenaustauscherharzen zusammenzubringen, von denen mindestens eines ein sulfonisches Harz und das andere (oder die anderen) ein Chelatharz ist, wobei diese Harze in der Säure- oder Ammoniumform verwendet werden.

2. Verfahren nach Anspruch 1, dadurch gekennzeichnet, daß jedem Harz ein Polystyrol-Divenylbenzol-Copolymeres zugrundeliegt.

3. Verfahren nach Anspruch 1 oder 2, dadurch gekennzeichnet, daß das Zusammenbringen des zu reinigenden DMSO mit den Austauscherharzen bei einer Temperatur von 19 bis 80 °C stattfindet.

4. Verfahren nach Anspruch 3, dadurch gekennzeichnet, daß die Temperatur zwischen 20 und 50 °C liegt.

## Claims

1. Process for purification of a dimethyl sulphoxide (DMSO) having a water content lower than or equal to 0.15 % by weight relative to the total weight, in order to decrease its content of alkali and alkaline-earth metal and metal cations, characterized in that it consists in placing the DMSO to be purified in contact with at least two cation exchange resins at least one of which is a resin of sulphonic type and the other (or the others) is of chelating type, these resins being employed in the acidic or ammonium form.

2. Process according to Claim 1, characterized in that each resin is based on a polystyrene-divinylbenzene copolymer.

3. Process according to Claim 1 or 2, characterized in that the contact of the DMSO to be purified with the exchange resins takes place at a temperature of 19 to 80°C.

4. Process according to Claim 3, characterized in that the temperature is between 20 and 50°C.
